# EUROPEAN PATENT SPECIFICATION

(11) **EP 0 510 772 B1**
(45) Date of publication and mention of the grant of the patent: **06.12.1995**
(21) Application number: 92201130.9
(22) Date of filing: 21.04.1992
(51) Int. Cl.: B01J 21/06, C07C 1/04

(54) **Process for the preparation of a catalyst or catalyst precursor**
Verfahren zur Herstellung von einem Katalysator oder von einem Katalysatorvorläufer
Procédé de préparation d'un catalyseur ou d'un précurseur de catalyseur

(30) Priority: 23.04.1991 GB 9108656
(43) Date of publication of application: 28.10.1992
(73) Proprietor: SHELL INTERNATIONALE RESEARCH MAATSCHAPPIJ B.V., 2596 HR Den Haag (NL)
(72) Inventor: Reinalda, Donald, NL-1031 CM Amsterdam (NL); Blankenstein, Paul, NL-1031 CM Amsterdam (NL)

(56) References cited:
- EP-A- 0 152 599
- EP-A- 0 244 301
- EP-A- 0 309 048
- EP-A- 0 428 223
- FR-A- 2 374 956
- US-A- 4 388 222

## Description

The present invention relates to a process for the preparation of a catalyst or catalyst precursor, in particular to the preparation of a zirconia-based catalyst or catalyst precursor.

Japanese patent application publication No. JP-A-63 063788 discloses a process for the preparation of a catalyst comprising a zirconium oxide/hydroxide carrier on which is deposited cobalt as a catalytically active component. The carrier is prepared by the addition of an alkali to an aqueous solution of a zirconium salt, for example zirconium chloride or zirconium nitrate. The resulting precipitate is washed, filtered and dried or burnt to yield the zirconium oxide/hydroxide carrier. The carrier is immersed in an aqueous solution of cobalt nitrate to deposit cobalt on the carrier. JP-A-63 063788 discloses that the resulting catalyst may be used in a direct process to obtain a middle-cut hydrocarbon fraction from a gaseous mixture of carbon monoxide and hydrogen.

The process disclosed in JP-A-63 063788 for the preparation of the carrier suffers the disadvantage that many steps are involved. The need for such a number of steps is undesirable for the preparation of a catalyst or catalyst carrier on a commercial scale. Further, the product of the process is a precipitate or powder which has only a limited number of applications as a support or carrier for a catalyst.

European patent application publication No. 0 244 301 (EP-A-0 244 301) discloses catalysts based on zirconium oxide for the treatment of sulphur-containing industrial gases. The catalysts are prepared by a process in which zirconium oxide, optionally in combination with an oxide of titanium or cesium, is connected with a solvent and an acid. The resulting mixture is extruded, dried and calcined. The resulting products had a structure with a low surface area.

Most surprisingly, it has now been found possible to prepare a zirconia-based catalyst or catalyst precursor in a simple process by means of extrusion, which process is very suitable for use on a commercial scale.

Accordingly, the present invention provides a process for the preparation of a zirconia-based catalyst or catalyst precursor comprising a carrier consisting substantially or wholly of zirconia, comprising mulling a mixture of zirconia and/or a zirconia precursor and a solvent, which mixture has a solids content of from 20 to 60% by weight, and extruding the mixture.

In the process of the present invention, a mixture comprising zirconia and/or a zirconia precursor and a solvent is mulled. The zirconia precursor may be any suitable compound. Suitable compounds to act as precursors for the zirconia include salts derived from organic acids, for example zirconium acetate, zirconium ethanoate, zirconium propionate and zirconium benzoate, and inorganic salts, for example zirconium chloride, zirconium bromide, zirconium iodide, zirconium fluoride, zirconium nitrate, zirconium hydroxide, zirconium carbonate, zirconium sulphate and zirconium chlorate. Inorganic zirconium salts are preferred zirconia precursors, with zirconium hydroxide being particularly preferred.

The solvent may be any of the suitable solvents known in the art, for example water; alcohols, such as methanol, ethanol and propanol; ketones, such as acetone; aldehydes, such as proponal; and aromatic solvents, such as toluene. Preferably and most conveniently, the solvent is water.

In addition to zirconia and/or a zirconia precursor, the mixture may comprise one or more other refractory oxides or precursors for refractory oxides, for example silica, titania, alumina, or mixtures thereof. The refractory oxide may be present in an amount of up to 50 parts by weight per 100 parts by weight of zirconia present in the final catalyst or catalyst precursor, more preferably up to 25 parts by weight. If a refractory oxide is present in the mixture it is preferably silica.

Suitable silicas for inclusion in the mixture include silica gel, precipitated silica and pyrogenic silica. Precipitated silica is a preferred silica for inclusion in the mixture.

The zirconium, present as zirconia, in the catalyst or catalyst precursor product of the process of the present invention may be used as a catalytically active component in the use of the product. If desired, however, the mixture to be mulled may also comprise sources for one or more catalytically active components or sources for one or more promoter elements of the catalytically active component. Accordingly, the mixture may comprise a source for one or more elements selected from Groups IB, IIB, IIIB, IVB, VB, VIB, VIIB, VIII of the Periodic Table of Elements, or the Lanthamides and Actinides. Preferred catalytically active components are the elements in Group VIII of the Periodic Table. Sources of elements selected from iron, ruthenium, cobalt, rhenium, nickel, palladium, platinum, copper and zinc are especially preferred. Cobalt, iron and nickel are particularly preferred catalytically active elements, with cobalt being most preferred. The mixture may also advantageously comprise a source for an element in Group IVB of the Periodic Table, which elements find use as promoters, in particular titanium, together with, if desired, an additional source for zirconium.

The source of the one or more elements from the aforementioned group may be either soluble or insoluble in the solvent. Typical sources include salts derived from organic acids, for example acetates, benzoates, ethanoates and propionates; halides, for example chlorides, bromides, iodides and fluorides; and other salts, for example nitrates, oxides, hydroxides, carbonates and chlorates. Sources insoluble in the solvent are preferred. Hydroxides have been found to be particularly suitable sources for the catalytically active elements and the promoters.

To obtain strong extrudates, it is preferred to include in the mixture a basic component to act as a peptizing agent for the zirconia and/or zirconia precursor. The basic compound is preferably ammonia, an ammonia-releasing compound, an ammonium compound or an organic amine. Such basic compounds are removed upon calcination and are not retained in the extrudates to impair the catalytic performance of the final product. The basic compound is most preferably an organic amine or an ammonium compound. A most suitable organic amine is ethanol amine. It has been found most convenient to include in the mixture a compound of the required element in the aforementioned groups of the Periodic Table, to act as both a source for that element and as the basic compound. In particular, it is preferred to include a source of an element acting as a promoter as the basic compound, most preferably a basic compound of an element in Group IVB of the Periodic Table. A preferred group of basic compounds are the ammonium carbonates. Ammonium zirconium carbonate is a particularly preferred basic compound for inclusion in the mixture.

A basic compound of an element of the aforementioned groups may be used in addition to both one or more of the aforementioned sources of that element and a basic compound. However, it has been found possible to dispense with both a separate source for the desired element and an additional basic compound by using a basic compound of the desired element.

The amount of basic compound included in the mixture should be sufficient to peptize the zirconia and/or zirconia precursor present in the mixture. The amount of basic compound present in the mixture can be readily determined by measuring the pH of the mixture. During mulling the mixture should preferably have a pH in the range of from 8.0 to 11.5, preferably from 9.0 to 11.0.

It has been found that the extrusion of mixtures having a high pH is more difficult than the extrusion of mixtures having a pH in the range of from 7.0 to 9.0. Accordingly, to avoid extruding a mixture having a high pH, it may be preferred to reduce the pH of the mixture, after allowing sufficient time for the zirconia and/or zirconia precursor to be peptized by the basic compound, but before extrusion, to a pH value in the range of from 7.0 to 8.0. The reduction in pH may be effected by the addition of an acid. Suitable acids include both organic and inorganic acids. Suitable inorganic acids include aqueous solutions of hydrogen fluoride, hydrogen chloride and hydrogen bromide, nitric acid, nitrous acid and perchloric acid. Preferably, an organic acid is used, for example an alkanoic acid having from 1 to 12 carbon atoms, preferably from 1 to 6 carbon atoms, or a dicarboxylic acid having from 1 to 6 carbon atoms. Particularly preferred acids are the alkanoic acids, especially formic acid, acetic acid, propionic acid and butanoic acid. Acetic acid is most preferred.

To improve the flux properties of the mixture during extrusion a surface active agent or polyelectrolyte may be added to the mixture. The addition of the surface active agent results in a smoother extrudate texture and facilitates cutting of the extruded product. Further, the formation of micropores in the calcined catalytic material may be improved which may enhance the catalytic properties of the products. Suitable surface active agents include cationic surface active agents, for example fatty amines, quaternary ammonium compounds, aliphatic monocarboxylic acids, ethoxylated alkyl amines, polyvinyl pyridine, sulphoxonium, sulphonium, phosphonium and iodonium compounds; anionic surface active agents, for example alkylated aromatic compounds, acyclic monocarboxylic acids, fatty acids, sulphonated aromatic compounds, alcohol sulphates, ether alcohol sulphates, sulphated fats and oils and phosphonic acid salts; and nonionic surface active agents, for example polyethylene alkylphenols, polyoxyethylene alcohols, polyoxyethylene alkylamines, polyoxyethylene alkylamides, polyols and acetylenic glycols. The amount of surface active agent is typically from 0.5 to 8% by weight, preferably from 1 to 5% by weight, based on the weight of zirconia and/or zirconia precursor present in the mixture. A preferred surface active agent is sold under the trademark Nalco. The surface active agent may be added at any stage during the mulling of the mixture prior to extrusion.

In the process of the present invention, a mixture of the aforementioned components is mulled. In principle, it is possible to combine the components of the mixture in any order. However, it has been found advantageous to prepare and mull the mixture in the following manner. At the very least, the mixture comprises zirconia and/or a zirconia precursor and a solvent, which are first mixed together. If the mixture is to include a basic compound, it has been found advantageous to add the basic compound to the mixture after the zirconia and/or zirconia precursor and solvent have been combined, together with any other refractory oxides or refractory oxide precursors. In this way, the considerable take up of the basic compound into the pores of the solid material is avoided, resulting in improved extrudates. Basic compound confined within the pores of the solid material is prevented from fully peptizing the material, requiring the addition of further amounts of the basic compound for satisfactory peptization, or yielding extrudates having a lower crush strength. The resulting mixture of solid material, solvent and basic compound may be mixed and mulled. Thereafter, once the solid material has been satisfactorily peptized, the pH of the mixture may be reduced as hereinbefore described. If desired, a source, of one or more elements in the aforementioned groups of the Periodic Table may be added and the resulting mixture subjected to further mulling. A surface active agent, if desired, may be added at any time during the mulling, preferably just prior to a final period of mulling.

Typically, the mixture is mulled for a period of from 10 to 120 minutes, preferably from 15 to 90 minutes. During the mulling process, energy is input into the mixture by the mulling apparatus. The rate of energy input into the mixture is typically from 0.05 to 50 Wh/min/kg, preferably from 0.5 to 10 Wh/min/kg. The mulling process may be carried out over a broad range of temperature, preferably from 15 to 50 °C. As a result of the energy input into the mixture during the mulling process, there will be a rise in temperature of the mixture during the mulling. The mulling process is conveniently carried out at ambient pressure. Any suitable, commercially available mulling machine may be employed.

Once the mulling process has been completed, the resulting mixture is then extruded. Extrusion may be effected using any conventional, commercially available extruder. In particular, a screw-type extruding machine may be used to force the mixture through orifices in a suitable dieplate to yield extrudates of the desired form. The strands formed upon extrusion may be cut to the desired length.

The extrudates may have the form of cylinders, for example, a hollow cylinder, or may have a form which is multilobed or twisted multilobed in cross section, or take any other form known in the art. The process of the present invention has been found to be particularly suitable for forming trilobe extrudates. Typically, the extrudates have a nominal diameter of from 0.5 to 5 mm, preferably from 1 to 3 mm.

After extrusion, the extrudates are dried. Drying may be effected at an elevated temperature, preferably up to 800 °C, more preferably up to 300 °C. The period for drying is typically up to 5 hours, preferably from 30 minutes to 3 hours.

Preferably, the extrudates are calcined after drying. Calcination is effected at an elevated temperature, preferably up to 1000 °C, more preferably from 200 °C to 1000 °C, most preferably from 300 °C to 800 °C. Calcination of the extrudates is typically effected for a period of up to 5 hours, preferably from 30 minutes to 4 hours.

Once prepared, the extrudates may be subjected to a deposition stage in which sources of one or more catalytically active elements or promoter elements are deposited on the extrudates. The sources may be of any of the elements in the groups of the Periodic Table as discussed herinbefore. In cases in which the original mixture comprised a source for a given element, the deposition of a further source for that element may be effected to increase the loading of the element on the extrudates.

Deposition of a source of a catalytically active element or a promoter element on the extrudates may be effected by any of the techniques known in the art, for example precipitation. If deposition of sources of a plurality of elements on the extrudate is to be effected, the deposition may be carried out in either one or a plurality of stages. The order of deposition of the plurality of sources of the elements is largely a matter of choice and convenience. However, the preferred order is first to deposit a promoter source on the extrudates and secondly to deposit a source of a catalytically active element.

A preferred technique for the deposition is impregnation. Impregnation may be effected by contacting the extrudates with a compound of the desired element in the presence of a liquid, preferably in the form of a solution of a compound of the element. Suitable liquids for use in the impregnation include both organic and inorganic liquids, water being a most convenient and preferred liquid. Suitable compounds of the desired element include both organic and inorganic compounds, with a preference for compounds which are soluble in the selected solvent. Preferably, the compounds are inorganic compounds. Nitrates are most preferred compounds.

The extrudates are most conveniently contacted with the compound of the desired element by immersion in the liquid. Preferably, the extrudates are immersed in a sufficient volume of liquid so as to just fill the volume of pores in the extrudates.

If the impregnation is conducted in a single stage, the extrudates are contacted simultaneously with a compound of each of the desired elements in the presence of the liquid. Preferably, the extrudates are immersed in an aqueous solution of nitrates of the desired elements. If the impregnation is conducted in a plurality of stages, the extrudates are contacted in a first stage with a compound of one of the desired elements in the presence of a liquid and in a subsequent stage with a compound of a further desired element in the presence of a liquid. The liquid may be the same or different in the stages; most conveniently the same.

After the impregnation, if in a single stage, or after each impregnation in a multi-stage impregnation, the extrudates are dried. The conditions under which the extrudates are dried are those as hereinbefore described. Preferably, after the or each drying process, the extrudates are calcined, the calcination conditions being those as hereinbefore described.

The catalytically active element may be present in the product in an amount of from 1 to 100 parts by weight, preferably 10 to 50 parts by weight, per 100 parts by weight of zirconia. The promoter, if present, may be present in an amount of from 1 to 60 parts by weight, preferably from 2 to 40 parts by weight, per 100 parts by weight of zirconia.

In a further aspect, the present invention provides a zirconia-based catalyst or catalyst precursor comprising a carrier consisting substantially or wholly of zirconia, having a surface area of 136 m²/g, obtainable by a process as hereinbefore described.

The products of the process of this invention may be applied in any process in which a zirconia-based catalyst can be used or is required. In particular, products of the process are particularly advantageous when comprising elements, optionally with one or more promoters, that are active, after reduction, as catalysts in the preparation of hydrocarbons from a mixture of carbon monoxide and hydrogen; the so-called Fischer-Tropsch synthesis. Of particular use in the Fischer-Tropsch synthesis are the products of this process comprising iron, nickel or cobalt as the catalytically active component. Products comprising cobalt are especially useful.

The products of the process of this invention may be reduced by contact with a hydrogen-containing gas at elevated temperature and pressure.

Thereafter, the resulting catalyst may be contacted with a mixture of carbon monoxide and hydrogen at an elevated temperature and pressure. Typically, the reaction is effected at a temperature in the range of from 125 to 350 °C, preferably from 175 to 250 °C. The reaction pressure is typically in a range from 5 to 100 bar, preferably from 12 to 50 bar. The hydrogen/carbon monoxide molar ratio in the feed gas is typically greater than 1.5, preferably between 1.75 and 2.25. Unconverted hydrogen and carbon monoxide may be recycled to again contact the catalyst. In such an arrangement, the molar ratio of hydrogen to carbon monoxide in the gas actually contacting the catalyst may be considerably lower than that of the feed gas for example in the range of from 0.9 to 1.3, preferably about 1.1.

The process of the present invention is further described in the following illustrative example. In the example, values for the loss on ignition are quoted on the basis of water lost upon heating the sample to a temperature in the range of from 550 to 600 °C. The extrusion process in the following example was conducted using a 1" Bonnot extruder having a 1.7 mm Delrin trilobe matrix dieplate insert yielding straight trilobe extrudates having a nominal diameter of 1.7mm. Calcination was conducted at the temperature and for the duration quoted in an atmosphere of air.

### Example

### Extrudate Preparation

A mixture comprising zirconium hydroxide (Zr(OH)₄, ex Magnesium Electron, 40 %wt equivalent of ZrO₂, 50g; Zr(OH)₄, ex Magnesium Electron, 47 %wt equivalent of ZrO₂, 100g), ammonium zirconium carbonate (Bacote 20, 20 %wt equivalent of ZrO₂, 40g) and silica (precipitated silica having an average particle size 50 »m, surface area 450 m²/g and a loss on ignition of 14 %wt, 29g) was mulled for a period of about 40 minutes. Polyelectrolyte (Nalco) (2g as a 2 %wt aqueous solution) was added and the resulting mixture mulled for a further 5 minutes, yielding a mixture having a loss on ignition of 53.8% and a pH of 8.4.

The resulting mixture was extruded using a 1" Bonnot extruder having a 1.7 mm Delrin trilobe dieplate insert to yield trilobe extrudates. The resulting extrudates were dried at a temperature of 120 °C and calcined at a temperature of 530 °C for 60 minutes. The resulting extrudates had a high crush strength, a pore volume (H₂O) of 0.257 ml/g and a surface area of 136 m²/g. The extrudates comprised 75.6 %wt (± 7.6) zirconia and 22.7 %wt (± 2.3) silica.

### Catalyst Preparation

Cobalt nitrate (Co(NO₃)₂).6H₂O, 16.7g) was melted in an oven by heating to a temperature of 84 °C to yield an aqueous solution of cobalt nitrate (10.0 ml). Extrudates (39.13g), as prepared above, were impregnated with cobalt by immersion in the aqueous solution of cobalt nitrate at 84 °C. The impregnated extrudates were dried for 30 minutes at a temperature of 60 °C. Finally, the extrudates were calcined for a period of 60 minutes at 500 °C.

## Claims

1. A process for the preparation of a zirconia-based catalyst or catalyst precursor comprising a carrier consisting substantially or wholly of zirconia, comprising mulling a mixture of zirconia and/or a zirconia precursor and a solvent, which mixture has a solids content of from 20 to 60% by weight, and extruding the mixture.

2. A process according to claim 1, wherein the mixture comprises a zirconia precursor, preferably a zirconium salt, more preferably zirconium hydroxide.

3. A process according to either of claims 1 or 2, wherein the mixture comprises a basic compound, preferably selected from ammonia, an ammonia-releasing compound, an ammonium compound or an organic amine, more preferably ethanoloamine or an ammonium zirconium compound especially ammonium zirconium carbonate.

4. A process according to any preceding claim, wherein the pH of the mixture is in the range of from 9.0 to 11.0.

5. A process according to claim 4, wherein the pH is reduced to a value in the range of from 7.0 to 9.0 prior to extrusion.

6. A process according to claim 5, wherein the pH is reduced by the addition of acetic acid to the mixture.

7. A process according to any preceding claim, wherein the mixture comprises another refractory oxide or refractory oxide precursor, present in an amount of up to 50 parts by weight per 100 parts by weight of zirconia present in the final product.

8. A process according to any preceding claim, wherein the mixture comprises a source of a catalytically active element or a promoter, preferably a source of an element selected from the elements in Group VIII of the Periodic Table, more preferably a source of cobalt, iron or nickel, especially cobalt.

9. A process according to any preceding claim, further comprising depositing on the resulting extrudates of a source of a catalytically active element or a promoter, preferably selected from the elements in Group VIII of the Periodic Table, more preferably cobalt, iron or nickel.

10. A zirconia-based catalyst or catalyst precursor comprising a carrier consisting substantially or wholly of zirconia, having a surface area of 136 m²/g, obtainable by a process according to any one of claims 1 to 9.

11. The use of a catalyst or catalyst precursor as claimed in claim 10 in a process for the preparation of hydrocarbons from a mixture of carbon monoxide and hydrogen.

## Patentansprüche

1. Verfahren zur Herstellung eines auf Zirkonerde basierenden Katalysators oder Katalysatorvorläufers, der einen weitgehend oder vollkommen aus Zirkonerde bestehenden Trägerstoff enthält, wobei man eine Mischung aus Zirkonerde und/oder einem Zirkonerdevorläufer und einem Lösungsmittel, welche einen Feststoffgehalt von 20 bis 60 Gew.-% aufweist, im Kollergang zermahlt und die Mischung extrudiert.

2. Verfahren nach Anspruch 1, wobei die Mischung einen Zirkonerdevorläufer, vorzugsweise ein Zirkoniumsalz, und besonders bevorzugt Zirkoniumhydroxid enthält.

3. Verfahren nach Anspruch 1 oder 2, wobei die Mischung eine vorzugsweise aus Ammoniak, einer Ammoniak freisetzenden Verbindung, einer Ammoniumverbindung oder einem organischen Amin, besonders bevorzugt Ethanolamin oder eine Ammonium-Zirkonium-Verbindung, insbesondere Ammonium-zirkonium-carbonat, ausgewählte basische Verbindung enthält.

4. Verfahren nach einem der vorhergehenden Ansprüche, wobei der pH-Wert der Mischung im Bereich von 9,0 bis 11,0 liegt.

5. Verfahren nach Anspruch 4, wobei man den pH-Wert vor der Extrusion auf einen Wert im Bereich von 7,0 bis 9,0 reduziert.

6. Verfahren nach Anspruch 5, wobei man den pH-Wert durch Zusatz von Essigsäure zu der Mischung reduziert.

7. Verfahren nach einem der vorhergehenden Ansprüche, wobei die Mischung ein weiteres feuerfestes Oxid oder einen Vorläufer eines feuerfesten Oxids enthält, welches/r in einer Menge von bis zu 50 Gewichtsteilen je 100 Gewichtsteile der im Endprodukt vorhandenen Zirkonerde vorliegt.

8. Verfahren nach einem der vorhergehenden Ansprüche, wobei die Mischung eine Quelle für ein katalytisch aktives Element oder einen Promotor, vorzugsweise eine Quelle eines aus den Elementen der Gruppe VIII des Periodensystems ausgewählten Elementes, besonders bevorzugt eine Cobalt-, Eisen- oder Nickel- und insbesondere eine Cobaltquelle, enthält.

9. Verfahren nach einem der vorhergehenden Ansprüche, das weiterhin das Anscheiden einer Quelle eines vorzugsweise aus den Elementen der Gruppe VIII des Periodensystems, besonders bevorzugt Cobalt, Eisen oder Nickel, ausgewählten katalytisch aktiven Elementes oder eines Promotors auf den erhaltenen Extrudaten umfaßt.

10. Katalysator oder Katalysatorvorläufer, der auf Zirkonerde basiert einen weitgehend oder vollkommen aus Zirkonerde bestehenden Trägerstoff mit einer Oberfläche von 136 m²/g enthält, erhältlich durch ein Verfahren nach einem der Ansprüche 1 bis 9.

11. Verwendung eines Katalysators oder Katalysatorvorläufers nach Anspruch 10 in einem Verfahren zur Herstellung von Kohlenwasserstoffen aus einer Mischung aus Kohlenmonoxid und Wasserstoff.

## Revendications

1. Procédé de préparation d'un précurseur de catalyseur ou d'un catalyseur à base de zircone, comprenant un support ou véhicule constitué essentiellement ou totalement de zircone, caractérisé en ce que l'on broie intimement un mélange de zircone et/ou d'un précurseur de zircone et d'un solvant, mélange qui possède une teneur en solides de 20 à 60% en poids, et on extrude le mélange.

2. Procédé suivant la revendication 1, caractérisé en ce que le mélange comprend un précurseur de zircone, de préférence un sel de zirconium, plus avantageusement l'hydroxyde de zirconium.

3. Procédé suivant l'une quelconque des revendications 1 et 3, caractérisé en ce que le mélange comprend un composé basique, choisi, de préférence, parmi l'ammoniac, un composé libérant de l'ammoniac, un composé d'ammonium et une amine organique, plus avantageusement l'éthanolamine ou un composé d'ammonium et de zirconium, plus spécialement le carbonate d'ammonium-zirconium.

4. Procédé suivant l'une quelconque des revendications précédentes, caractérisé en ce que le pH du mélange varie de 9,0 à 11,0.

5. Procédé suivant la revendication 4, caractérisé en ce que le pH est réduit jusqu'à une valeur qui fluctue de 7,0 à 9,0 avant l'extrusion.

6. Procédé suivant la revendication 5, caractérisé en ce que la valeur du pH est réduite par l'addition d'acide acétique au mélange.

7. Procédé suivant l'une quelconque des revendications précédentes, caractérisé en ce que le mélange comprend un autre oxyde réfractaire ou un autre précurseur d'oxyde réfractaire, présent en une proportion allant jusqu'à 50 parties en poids par 100 parties en poids de zircone présente dans le produit final.

8. Procédé suivant l'une quelconque des revendications précédentes, caractérisé en ce que le mélange comprend une source d'un élément catalytiquement actif ou d'un promoteur, de préférence, une source d'un élément choisi parmi les éléments du groupe VIII du tableau périodique, plus avantageusement, une source de cobalt, de fer ou de nickel, plus spécialement de cobalt.

9. Procédé suivant l'une quelconque des revendications précédentes, caractérisé en ce qu'il comprend, en outre, le dépôt, sur les extrudats ainsi obtenus, d'une source d'un promoteur ou d'un élément catalytiquement actif, que l'on choisit, de préférence, parmi les éléments du groupe VIII du tableau périodique, de préférence, le cobalt, le fer ou le nickel.

10. Précurseur de catalyseur ou catalyseur à base de zircone, comprenant un support ou véhicule essentiellement constitué ou totalement constitué de zircone, possédant une surface spécifique de 136 m²/g, que l'on peut obtenir par mise en oeuvre d'un procédé suivant l'une quelconque des revendications 1 à 9.

11. Utilisation d'un précurseur de catalyseur ou d'un catalyseur suivant la revendication 10 dans un procédé de préparation d'hydrocarbures au départ d'un mélange de monoxyde de carbone et d'hydrogène.
